# EUROPEAN PATENT APPLICATION

(11) **EP 3 187 165 A1**
(43) Date of publication of application: **05.07.2017**
(21) Application number: 16205210.4
(22) Date of filing: 20.12.2016
(51) Int. Cl.: A61K 8/34, A61K 8/37, A61Q 19/10, A61K 8/04, A01N 25/16, A01N 31/02, A01N 31/04, A01N 37/02, A01N 37/12, A61L 2/18

(54) **FOAMING HAND SANITIZERS**

(30) Priority: 29.12.2015 US 201514982088
(71) Applicant: The Dial Corporation, Scottsdale, AZ 85255 (US)
(72) Inventor: LUCIOW, Chris, Valley, AZ 85253, Paradise (US); STAMPER, Aurora, AZ 85085, Phoenix, (US); FERNANDEZ, James A., AZ 85023, Phoenix, (US); CONWAY, Mary J, AZ 85022, Phoenix, (US)
(74) Representative: Semrau, Markus

(57) **Abstract**

Hand sanitizers and methods for producing and using the same are provided. In an embodiment, a hand sanitizer comprises an alkyl monoalcohol at from about 50 to about 95 weight percent, based on the total weight of the hand sanitizer, where the monoalcohol has from 1 to 3 carbon atoms. The hand sanitizer also comprises a glycerin alkyl ether at from about 0.1 to about 5 weight percent, a glycerin alkyl ester at from about 0.1 to about 3 weight percent, an aromatic alcohol at from about 0.1 to about 3 weight percent, and a surfactant at from about 0.5 to about 10 weight percent. All weight percents are based on the total weight of the hand sanitizer, and the surfactant comprises a fluorosurfactant, a silicone based surfactant, or a combination thereof.

## Description

### Technical field

The technical field generally relates to foaming hand sanitizers and methods to produce and use the same, and more particularly relates to foaming hand sanitizers with alcohol and an enhanced antimicrobial effect, and methods to produce and use the same.

### Background

One of the primary methods of reducing the spread of germs and diseases is the use of proper hand care and sanitation. This is especially true in industries or areas where bacterial contamination is particularly problematic, such as health care and food and beverage industries. Hand washing with soap and water is commonly used, but hand sanitizers that do not need to be washed off have become popular as well. The user applies the hand sanitizer onto the hands and spreads it around such that the hand sanitizer contacts most or all of the skin on the hands. Hand sanitizers are available in lotions, gels, or foams, and many include antimicrobial agents. One such antimicrobial agent is alcohol, and alcohol-based hand sanitizing products are known and in use. Alcohol-based sanitizers can be found in hospitals and other healthcare environments, the workplace, and in everyday home use. Hand sanitizers with alcohol of about 50% or more have a very rapid antimicrobial effect, but the alcohol may not have a persistent antimicrobial effect. Alcohol kills many microbes on contact, but alcohol may evaporate and leave the hands or other surfaces unprotected. Some components may be added to the hand sanitizer to help slow the evaporation of alcohol, but alcohol will still evaporate. Alcohol is very effective against many bacteria, but alcohol is not effective against all microbes. For example, alcohol has limited efficacy against some viruses, such as many noroviruses and the rabies virus. As such, some illnesses may be caused by microbes that survive exposure to alcohol.

Accordingly, it is desirable to provide hand sanitizers with enhanced antimicrobial efficacy, and methods for producing and using the same. In addition, it is desirable to provide hand sanitizers that provide increased antimicrobial persistence over hand sanitizers using alcohol as the sole antimicrobial ingredient, and methods of producing and using the same. Other desirable features and characteristics of the present embodiment will become apparent from the subsequent detailed description and the appended claims, taken in conjunction with the accompanying drawings and this background of the invention.

### Brief summary

Hand sanitizers and methods for producing and using the same are provided. In an embodiment, a hand sanitizer comprises an alkyl monoalcohol at from about 50 to about 95 weight percent, based on the total weight of the hand sanitizer, where the monoalcohol has from 1 to 3 carbon atoms. The hand sanitizer also comprises a glycerin alkyl ether at from about 0.1 to about 5 weight percent, a glycerin alkyl ester at from about 0.1 to about 3 weight percent, an aromatic alcohol at from about 0.1 to about 3 weight percent, and a surfactant at from about 0.5 to about 10 weight percent. All weight percents are based on the total weight of the hand sanitizer, and the surfactant comprises a fluorosurfactant, a silicone based surfactant, or a combination thereof.

In another embodiment, a method is provided for using a hand sanitizer. An effective amount of the hand sanitizer is applied to the hands. The hand sanitizer comprises an alkyl monoalcohol at from about 50 to about 95 weight percent, a glycerin alkyl ether at from about 0.1 to about 5 weight percent, a glycerin alkyl ester at from about 0.1 to about 3 weight percent, an aromatic alcohol at from about 0.1 to about 3 weight percent, and a surfactant at from about 0.5 to about 10 weight percent, where all weight percentages are based on the total weight of the hand sanitizer. The alkyl monoalcohol has from 1 to 3 carbon atoms, and the surfactant comprises a fluorosurfactant, a silicone based surfactant, or a combination thereof.

In yet another embodiment, a method is provided for producing a hand sanitizer. An alkyl monoalcohol is combined with a glycerin alkyl ether, a glycerin alkyl ester, an aromatic alcohol, and surfactant. The alkyl monoalcohol is added at from about 50 to about 95 weight percent, the glycerin alkyl ether is added at from about 0.1 to about 5 weight percent, the glycerin alkyl ester is added at from about 0.1 to about 3 weight percent, the aromatic alcohol is added at from about 0.1 to about 3 weight percent, and the surfactant is added at from about 0.5 to about 10 weight percent, where all weight percentages are based on the total weight of the hand sanitizer. The alkyl monoalcohol has from 1 to 3 carbon atoms, and the surfactant comprises a fluorosurfactant, a silicone based surfactant, or a combination thereof.

### Brief description of the drawings

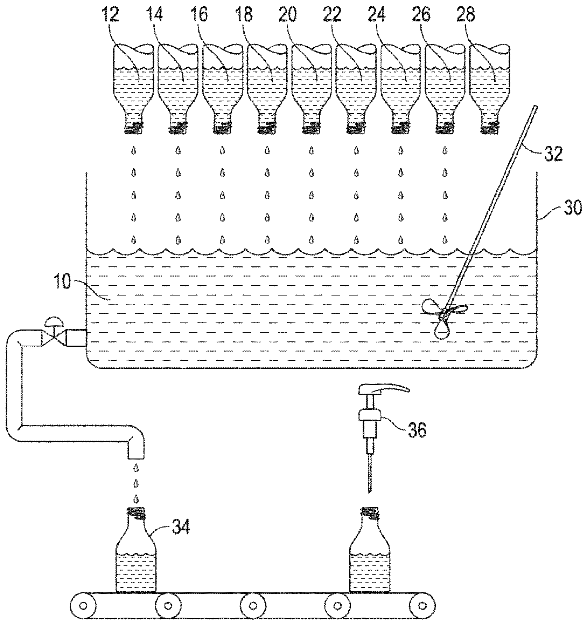

The present embodiments will hereinafter be described in conjunction with the drawing figure, wherein:
The FIGURE is a schematic representation of an exemplary embodiment of a hand sanitizer and a method of producing the same.

### Detailed description

The following detailed description is merely exemplary in nature and is not intended to limit the various embodiments of the hand sanitizer and methods of preparing the same, or the application and uses of the hand sanitizers. Furthermore, there is no intention to be bound by any theory presented in the preceding background or the following detailed description.

Reference is made to the FIGURE. The various embodiments contemplated herein relate to hand sanitizers 10 with enhanced antimicrobial activity, where "enhanced" antimicrobial activity, as used herein, means antimicrobial activity that is greater than the antimicrobial activity of a hand sanitizer 10 that uses alcohol as the only antimicrobial agent.

The hand sanitizer 10 includes an alkyl monoalcohol 12 at from about 50 weight percent to about 95 weight percent in an exemplary embodiment, but in alternate embodiments the hand sanitizer 10 may include an alkyl monoalcohol 12 at from about 60 to about 95 weight percent or from about 75 to about 95 weight percent, where the weight percent is based on the total weight of the hand sanitizer 10. The percentage of all components of the hand sanitizer 10 described herein are weight percent, based on the total weight of the hand sanitizer 10, unless otherwise specified. Alkyl monoalcohols 12 are a very effective antimicrobial agent when present at concentrations of about 50 weight percent, or about 60 weight percent, or about 75 weight percent or more, and there appears to be little to no genetic mutation that produces microbes that are resistant to the antimicrobial effect of alkyl monoalcohols 12. Alkyl monoalcohols 12 produce a very rapid antimicrobial effect, and may kill about 99.9 percent of bacteria on a person's hands within about 30 seconds after application when applied at sufficient concentrations, and higher kill rates may be achieved with longer exposure time. Alkyl monoalcohols 12 kill many bacteria that are resistant to various antibiotics, and alkyl monoalcohols 12 also kill many types of viruses and fungi. However, alkyl monoalcohols 12 are not effective against all bacteria and virus.

The term "alkyl monoalcohol," as used herein, means alkyl monoalcohols 12 that include from 1 to 3 carbons and one hydroxyl group. As such, the term "alkyl monoalcohol," as used herein, means one or more of methanol, ethanol, isopropanol, or n-propanol. Other alcohols may also be present in the hand sanitizer 10, such as a butyl alcohol, 1,2 dipropanol, 1,3 dipropanol, etc., but the alkyl monoalcohol 12 is present at the concentration described above. The alkyl monoalcohols 12 are relatively volatile, and may evaporate after application to the hands or other surfaces that are in the open air. Alkyl monoalcohols 12 will remain in a solution when stored in a closed container, so refrigeration is generally not needed for the hand sanitizer 10. Certain formulations of hand sanitizers 10 may limit or slow the evaporation of the alkyl monoalcohols 12 after being placed in an open area, such as on the hands.

In some embodiment, the hand sanitizer 10 may be a foaming type of hand sanitizer 10. A thickener 14 may or may not be present in foaming types of hand sanitizers 10, and the viscosity of foaming type hand sanitizers 10 may be different than the viscosity of the gel type hand sanitizers 10, as described below. The foaming hand sanitizer 10 may include a surfactant 16 that produces a foam with an alkyl monoalcohol 12, such as many silicone polymer surfactants and/or fluorosurfactants. The gel-type hand sanitizers 10 may also include a surfactant 16. In an exemplary embodiment, the surfactant 16 is present in the hand sanitizer 10 at from about 0.05 to about 8 weight percent, based on the total weight of the hand sanitizer 10. In alternate embodiments, the surfactant 16 may be present in the hand sanitizer 10 from about 0.05 to about 5 weight percent, or from about 0.05 to about 2 weight percent. Examples of suitable surfactants 16 include, but are not limited to, one or more of dimethicone copolyols including but not limited to PEG 10 dimethicone and PEG-12 dimethicone, polysiloxane-polyether copolymers including but not limited to bis PEG polypropylene glycol (PPG) 20/20 dimethicone, dimethicones and dimethicone copolyols with polyethyleneoxide (PEO) and polypropylene oxide (PPO) side chains, polyoxyethylene ethers, polyoxyethylene esters, polyoxyethylene fatty acids, polyoxyethylene sulfated acids, sulfosuccinates, polysiloxane, sorbitan fatty acid esters, polyoxamers, linear PEG 8-PEG-12 dimethicones, dimethicone copolymers including but not limited to PEG/PPG 18/18, 17/18, 4112, 35/65, and combinations thereof. In some embodiments, the surfactant 16 is one or more of a fluorosurfactant and a silicone based surfactant, such as a silicone polymer surfactant. The foaming type of hand sanitizer 10 may have a viscosity of about 1 to about 1,000 centipoise in an exemplary embodiment, but in alternate embodiments the viscosity may be from about 3 to about 800 or from about 5 to about 500 centipoise.

The hand sanitizer 10 may include antimicrobial agents in addition to the alkyl monoalcohol 12, where the additional antimicrobial agents may impart an enhanced antimicrobial effect. The additional antimicrobial agents and other components described below may be present in foaming or gel type hand sanitizers 10. In an exemplary embodiment, an "enhanced antimicrobial effect" means a bacterial kill rate for the hand sanitizer 10 that is greater than a bacterial kill rate for a test blank that consists of the alkyl monoalcohol 12 of the hand sanitizer in an aqueous solution, where the alkyl monoalcohol 12 is at the same concentration in the test blank and in the hand sanitizer 10 with the additional antimicrobial agents. An "enhanced antimicrobial effect" may also mean a more persistent antimicrobial effect, where the hand sanitizer 10 with the additional antimicrobial agents has an antimicrobial effect for a longer period of time than the test blank. Some or all of the additional antimicrobial agents may be less volatile than the alkyl monoalcohol 12 to provide a more persistent antimicrobial effect, and some or all of the additional antimicrobial agents may have a different type of antimicrobial activity that compliments the antimicrobial activity of the alkyl monoalcohol 12.

The hand sanitizer 10 may include one or more than one additional antimicrobial agents. One such antimicrobial agent is a glycerin alkyl ether 18, which may be present in the hand sanitizer 10 at from about 0.1 to about 5 weight percent in an exemplary embodiment. In alternate embodiments, the glycerin alkyl ether 18 may be present at from about 0.1 to about 3 weight percent or from about 0.1 to about 1 weight percent, based on the total weight of the hand sanitizer 10. The glycerin alkyl ether 18 may be a glycerin alkyl monoether in some embodiments, such that the glycerin alkyl ether 18 has the formula: (HO)-C-C(OH)-C-O-R, wherein R is an alkyl moiety. The alkyl moiety R may be a straight chain or branched in various embodiments. In an exemplary embodiment, the glycerin alkyl ether 18 is ethylhexylglycerin.

Another antimicrobial agent that may be present in the hand sanitizer 10, in addition to or in place of the glycerin alkyl ether 18 is a glycerin alkyl ester 20. The glycerin alkyl ester 20 may be present at from about 0.1 to about 3 weight percent in an exemplary embodiment, based on the total weight of the hand sanitizer 10. The glycerin alkyl ester 20 may be present at from about 0.1 to about 2 weight percent or from about 0.1 to about 1 weight percent in alternate embodiments. In some embodiments, the glycerin alkyl ester 20 is a glycerin alkyl monoester, such that the glycerin alkyl monoester has the formula (HO)-C-C(OH)-C-O-C(O)-R', wherein R' is an alkyl moiety. The alkyl moiety R' may be a straight chain or a branched alkyl in various embodiments. In one embodiment, the glycerin alkyl ester 20 is glyceryl caprylate.

Yet another antimicrobial agent that may be present in the hand sanitizer 10, in addition to on in place of the glycerin alkyl ether 18 and/or the glycerin alkyl ester 20, is an aromatic alcohol 22. The aromatic alcohol 22 may be present in the hand sanitizer 10 at from about 0.1 to about 3 weight percent in some embodiments, based on the total weight of the hand sanitizer 10, but the aromatic alcohol 22 may be present from about 0.1 to about 2 weight percent or from about 0.1 to about 1 weight percent in alternate embodiments. In an exemplary embodiment, the aromatic alcohol 22 is an aromatic alkyl monoalcohol, such that the aromatic alkyl monoalcohol has the formula C6H5-R"-(OH), wherein R" is an alkyl moiety. The alkyl moiety R" may be branched or straight in various embodiments. In an alternate embodiment, the aromatic alcohol 22 may be phenol. In one exemplary embodiment, the aromatic alcohol 22 is phenethyl alcohol.

Additional antimicrobial agents may be used in some embodiments, and certain antimicrobial agents may not be present. In an exemplary embodiment, 5-chloro-2-methyl-4-isothiazolin-3-one and 2-methyl-4-isothiazolin-3-one are not present in the hand sanitizer 10, such that 5-chloro-2-methyl-4-isothiazolin-3-one and 2-methyl-4-isothiazolin-3-one are present at from about 0 to about 0.0001 weight percent, based on the total weight of the hand sanitizer 10. Other antimicrobial agents that may not be present include triclosan, povidone-iodine, and quaternary ammonium salts, each of which may not be present such that the concentration of triclosan, povidone-iodine, and/or quaternary ammonium salts is from about 0 to about 0.0001 weight percent, based on the total weight of the hand sanitizer 10. However, in alternate embodiments, one or more of 5-chloro-2-methyl-4-isothiazolin-3-one, 2-methyl-4-isothiazolin-3-one, triclosan, povidone-iodine, and/or quaternary ammonium salts may be present, such as at from about 0.1 to about 5 weight percent, based on the total weight of the hand sanitizer 10.

The hand sanitizer 10 may optionally include several other components in various embodiments. For example, water 24 may be present at from about 5 to about 40 weight percent, based on the total weight of the hand sanitizer 10. Water 24 may be present at from about 5 to about 30 weight percent or from about 10 to about 30 weight percent or from about 15 to about 30 weight percent in various embodiments, based on the total weight of the hand sanitizer 10. Another optional ingredient includes one or more skin conditioners 26, which may be present at from about 0.1 to about 5 weight percent in an exemplary embodiment. In alternate embodiments the skin conditioner 26 may be present at from about 0.1 to about 2 weight percent, or from about 0.5 to about 2 weight percent, based on the total weight of the hand sanitizer 10. Several different compounds may serve as skin conditioners 26, wherein the skin conditioner 26 is a moisturizer that helps to soften skin or to treat dry skin. A skin conditioner 26 increases the ability of the skin to hold water, and this provides the skin with a layer of oil to prevent water loss and to lubricate the skin. Exemplary embodiments of skin conditioners 26 include, but are not limited to: octyl isononanoate; cetyl myristate; glyceryl dioleate; methyl laurate; PPG-9 laurate; soy stearyl; octyl palmitate; PPG-5 lanoate; lanolin; propylene glycol; glycerine; fatty acids; natural oils such as almond, mineral, canola, sesame, avocado, balm mint, cod liver, soybean, wheat germ, corn, peanut, vegetable, and olive; isopropyl myristate; myristyl alcohol; aloe vera; hydrolyzed silk protein; vitamin E; stearyl alcohol; isopropyl palmitate; sorbitol; amino acid complexes; polyethylene glycol; agarose; arginine PCA; fructose; glucose; glutamic acid; honey; lactose; maltose; methicone; phenyl trimethicone; trimyristin; stearyl stearate; synthetic wax; cholesterol; cystine; keratin; lecithin; egg yolk; glycine; PPG-12; retinol; salicylic acid; orotic acid; vegetable oil; and others.

The hand sanitizer 10 may also include fragrance 28 in some embodiments. As used herein, "fragrance" is a chemical compound that humans can detect by smell, where the fragrance is added to the hand sanitizer 10 for the purpose of its smell. The fragrance 28 may be present in the hand sanitizer 10 at from about 0 to about 5 weight percent, but the fragrance 28 may be present in the hand sanitizer 10 at from about 0.01 to about 5 weight percent, or from about 0.01 to about 3 weight percent, or from about 0.01 to about 0.5 weight percent in various embodiments, based on the total weight of the hand sanitizer 10. Many compounds may be added as a fragrance 28, including but not limited to: natural extracts, oils, resinoids, resins, and/or essences which may include one or more of many different constituents, such as orange, lemon, rose, nutmeg, lavender, musk, patchouli, balsam, sandalwood, pine, cedar, and cassia; 7-acetyl-1, 2, 3, 4, 5, 6, 7, 8-octahydro-1, 1, 6, 7-tetramethylnaphthalene, -ionone, β- ionone, γ-ionone, a-isomethylionone; methyl cedryl ketone; methyl dihydrojasmonate; methyl 1, 6, 10-trimethyl-2, 5, 9-cyclododecatrien-l-yl ketone; 4-acetyl-6-tert-butyl-1, 1-dimethylindane; hydroxyphenylbutanone; benzophenone; methyl β-naphthyl ketone; 6-acetyl-l, 1, 2, 3, 3, 5-hexamethylindane; 5-acetyl-3-isopropyl-1, 1, 2, 6-tetramethylindane; 1-dodecanal; 4-(4-hydroxy-4-methylpentyl)-3-cyclohexene-1-carboxaldehyde; 7-dimethyloctanal; 10-undecen-1-al; isohexenylcyclohexylcarboxaldehyde; formyl tricyclodecane; condensation products of hydroxycitronellal and methyl anthranilate; condensation products of hydroxycitronellal and indole; condensation products of phenylacetaldehyde and indole; 2-methyl-3-(para-tert-butylphenyl) propionaldehyde; ethylvanillin; heliotropin; hexylcinnamaldehyde; amylcinnamaldehyde; 2-methyl-2-(isopropylphenyl) propionaldehyde; coumarin; decalactone-γ; cyclopentadecanolide; 16-hydroxy-9-hexadecenolactone; 1, 3, 4, 6, 7, 8-hexahydro-4, 6, 6, 7, 8, 8-hexamethylcyclopenta-Y-2-benzopyran; β-naphthol methyl ether; ambroxan; 5-(2,2,3-trimethylcyclopent-3-enyl)-3-methylpentan-2-ol; 2-ethyl-4-(2, 2, 3-trimethyl-3-cyclopenten-l-yl)-2-buten-l-ol; caryophyllene alcohol; tricyclodecenyl propionate; tricyclodecenyl acetate; benzyl salicylate; cedryl acetate; tert-butyleyelohexyl acetate; and many other compounds.

The hand sanitizer 10 may be produced by combining the components described above. In an exemplary embodiment, the components described above are combined at the concentrations described above in a vessel 30 and mixed, such as with an agitator 32, but in-line mixers or other methods of mixing may also be used. In alternate embodiments, some of the components described above may be mixed together, and other components may be added and blended until the desired hand sanitizer 10 is produced. The hand sanitizer 10 may then be packaged in a container 34, and a pump 36 may be affixed to the container 34. The pump 36 may be a hand pump in some embodiments, but the pump 36 may also be electrically activated or otherwise activated to dispense the hand sanitizer 10 in alternate embodiments. In yet other embodiments, the container 34 includes a lid (not illustrated), where the lid may not include a pump 36 such that the hand sanitizer 10 is dispensed by pouring or squeezing the hand sanitizer 10 out of the container 34. In some embodiments, the pump 36 may combine air with the hand sanitizer 10 prior to the hand sanitizer 10 exiting the pump 36, such that the hand sanitizer 10 foams when dispensed.

The hand sanitizer 10 may be used by applying an effective amount of hand sanitizer 10 to the hands of a person, and spreading the hand sanitizer 10 over the surface of the skin. An effective amount is the amount of hand sanitizer 10 that kills at least about 90 percent of the bacteria on the surface where the hand sanitizer 10 is applied. In an exemplary embodiment, an effective amount of hand sanitizer 10 is about 0.005 grams per square centimeter of skin or other surface that the hand sanitizer 10 is spread over.

Table 1 below shows test data that illustrates the improved efficacy of hand sanitizers with antimicrobials (labelled "additive") other than ethanol. The test results in the Table 1 are based on a 2.0 milliliter (ml) dose of a test hand sanitizer, and all results are based on reducing the bacteria Serratia Marcescens ATCC 14756 after 1 treatment. Bacterial counts were converted into Log10 reduction values, where higher values indicate higher antibacterial efficacy.

**Table 1**

| Sample | % ethanol | Additive | Percent additive | Log₁₀ reduction |
|---|---|---|---|---|
| 1 | 62 | None | N/A | 1.73 |
| 2 | 70 | ethylhexylglycerin | 1.0 | 2.46 |
| 3 | 70 | glyceryl caprylate | 1.0 | 2.17 |

While at least one exemplary embodiment has been presented in the foregoing detailed description, it should be appreciated that a vast number of variations exist. It should also be appreciated that the exemplary embodiment or exemplary embodiments are only examples, and are not intended to limit the scope, applicability, or configuration of the application in any way. Rather, the foregoing detailed description will provide those skilled in the art with a convenient road map for implementing one or more embodiments, it being understood that various changes may be made in the function and arrangement of elements described in an exemplary embodiment without departing from the scope, as set forth in the appended claims.

## Claims

1. A hand sanitizer comprising:
an alkyl monoalcohol at from about 50 to about 95 weight percent, based on a total weight of the hand sanitizer, wherein the alkyl monoalcohol has from 1 to 3 carbon atoms;
a glycerin alkyl ether at from about 0.1 to about 5 weight percent, based on the total weight of the hand sanitizer;
a glycerin alkyl ester at from about 0.1 to about 3 weight percent, based on the total weight of the hand sanitizer;
an aromatic alcohol at from about 0.1 to about 3 weight percent, based on the total weight of the hand sanitizer; and
a surfactant at from about 0.5 to about 8 weight percent, based on the total weight of the hand sanitizer, wherein the surfactant comprises a fluorosurfactant, a silicone based surfactant, or a combination thereof.

2. The hand sanitizer of claim 1 wherein the alkyl monoalcohol comprises ethanol, isopropanol, or a combination thereof.

3. The hand sanitizer of claim 1 wherein the glycerin alkyl ether comprises ethylhexylglycerin.

4. The hand sanitizer of claim 1 wherein the glycerin alkyl ester comprises glyceryl caprylate.

5. The hand sanitizer of claim 1 wherein the aromatic alcohol comprises phenethyl alcohol.

6. The hand sanitizer of claim 1 wherein the hand sanitizer comprises the alkyl monoalcohol at from about 60 to about 95, preferably from 75 to about 95 weight percent, based on the total weight of the hand sanitizer.

7. The hand sanitizer of claim 1 further comprising water at from about 5 to about 40 weight percent.

8. The hand sanitizer of claim 1 further comprising a skin conditioner at from about 0.1 to about 2 weight percent, based on the total weight of the hand sanitizer.

9. The hand sanitizer of claim 1 wherein the hand sanitizer comprises the glycerin alkyl ether at from about 0.1 to about 3 weight percent, based on the total weight of the hand sanitizer.

10. The hand sanitizer of claim 1 wherein the hand sanitizer is free of 5-chloro-2-methyl-4-isothiazolin-3-one and 2-methyl-4-isothiazolin-3-one, such that 5-chloro-2-methyl-4-isothiazolin-3-one and 2-methyl-4-isothiazolin-3-one are present at a concentration of about 0 to about 0.001 weight percent, based on the total weight of the hand sanitizer.

11. A method of using a hand sanitizer comprising:
applying an effective amount of the hand sanitizer to a hand, wherein the hand sanitizer comprises:
an alkyl monoalcohol at from about 50 to about 95 weight percent, based on a total weight of the hand sanitizer, wherein the alkyl monoalcohol has from 1 to 3 carbon atoms;
a glycerin alkyl ether at from about 0.1 to about 5 weight percent, based on the total weight of the hand sanitizer;
a glycerin alkyl ester at from about 0.1 to about 3 weight percent, based on the total weight of the hand sanitizer;
an aromatic alcohol at from about 0.1 to about 3 weight percent, based on the total weight of the hand sanitizer; and
a surfactant at from about 0.5 to about 8 weight percent, based on the total weight of the hand sanitizer, wherein the surfactant comprises a fluorosurfactant, a silicone based surfactant, or a combination thereof.

12. A method of producing a hand sanitizer comprising:
combining an alkyl monoalcohol with a glycerin alkyl ether, a glycerin alkyl ester, an aromatic alcohol, and a surfactant wherein:
the alkyl monoalcohol is added at from about 50 to about 95 weight percent, based on a total weight of the hand sanitizer, and wherein the alkyl monoalcohol has from 1 to 3 carbon atoms;
the glycerin alkyl ether is added at from about 0.1 to about 5 weight percent, based on the total weight of the hand sanitizer;
the glycerin alkyl ester is added at from about 0.1 to about 3 weight percent, based on the total weight of the hand sanitizer;
the aromatic alcohol is added at from about 0.1 to about 3 weight percent, based on the total weight of the hand sanitizer; and
the surfactant is added at from about 0.5 to about 8 weight percent, based on the total weight of the hand sanitizer, wherein the surfactant comprises a fluorosurfactant, a silicone based surfactant, or a combination thereof.

13. The method of claim 12 further comprising:
packaging the hand sanitizer with a pump, wherein the pump combines air with the hand sanitizer prior to the hand sanitizer exiting the pump.
